# EUROPEAN PATENT SPECIFICATION

(11) **EP 0 548 050 B1**
(45) Date of publication and mention of the grant of the patent: **27.08.1997**
(21) Application number: 93103678.4
(22) Date of filing: 14.12.1989
(51) Int. Cl.: A61F 13/58, A61F 13/56

(54) **Fastening system for disposable diaper with disposability feature**
Verschluss für eine Wegwerfwindel zum Fixieren im verschmutzten Zustand
Système de fermature pour couche de bébé à jeter pourvue d'un élément jetable

(30) Priority: 20.12.1988 US 287746
(43) Date of publication of application: 23.06.1993
(62) Divisional of application: 89123131.8
(73) Proprietor: KIMBERLY-CLARK CORPORATION, Neenah Wisconsin 54957-0349 (US)
(72) Inventor: Roessler, Thomas Harold, Menasha, Wisconsin 54925 (US); Siebers, Bruce Michael, Appleton, Wisconsin 54915 (US); Popp, Robert Lee, Hortonville, Wisconsin 54944 (US); Fallen, Charles Rayburn, Neenah, Wisconsin 54956 (US)
(74) Representative: Grünecker, Kinkeldey, Stockmair & Schwanhäusser Anwaltssozietät

(56) References cited:
- WO-A-83/03754
- DE-A- 2 504 210
- NL-A- 7 103 602
- US-A- 3 150 664
- US-A- 4 681 581

## Description

The present invention relates to an absorbent article of the type as outlined in the preamble of claim 1.

A diaper of this type which can be rolled up into a condition for disposal is described in US-A-3 869 761. The diaper has a primary fastening means consisting of a pair of adhesively coated tabs arranged at one waistband portion of the diaper and adapted to be stuck to the diaper body if the diaper is attached to the body of the user. The diaper further has a secondary fastening means consisting of two further adhesively coated tabs which are covered in use of the diaper by the tabs of the primary fastening system. When the diaper is to be disposed, the tabs of the primary fastening system are to be torn off the diaper, thus exposing the adhesive coating of the tabs of the secondary fastening system to be attached to the body of the diaper if the diaper is in rolled up condition for disposal.

DE-A-2 504 210 describes a fastening system for disposable diapers. The fastening system includes a primary fastening system of the adhesive type for securing the diaper when worn. The fastening system includes laterally extending tapes provided with an adhesive layer, and with perforations for adhering of a portion of the tapes after the diaper is to be removed for disposal. By adhering of a portion of the tapes a further adhesive layer is exposed which is to be attached to the lateral sides of the diaper when in a rolled-up state for disposal.

Further fastening systems heretofore in use have included adhesive fastening, such as shown, in US-A-3 180 355; US-A-3 630 201; US-A-4 047 530; US-A-4 049 001; US-A-4 050 453 and FR-A-7 436 169. The fastening device generally comprises an adhesive tab attached to the outer (backing) sheet of the diaper at the rear portion and the active adhesive surface of the tab is stuck on to another portion of the backing sheet surface near to the front portion thereby closing the diaper. The adhesive system for the primary fastening system of the waistband around the wearer is susceptible to contamination of either the adhesive tab surface or the cover surface to which the tab is applied. Recently, mechanical closure systems have been devised using hook (e.g. "mushroom") and loop (e.g. Velcro®) fasteners for closure of the waistband as set forth by FR-A-2 594 650 and EP-A-0 276 970.

It will therefore, be appreciated that there has been a significant need for a fastening system for a disposable article which is easy in design and easy in use, and which is able to securely seal a soiled absorbent article after use in a rolled or folded condition for disposal.

An absorbent article fulfilling this need is described in claim 1.

The secondary fastening means in accordance with the present invention is easy in design, easy to manufacture and easy in use. The secondary fastening means is easy to be identified by the user as to be suitable to hold the diaper in disposal condition, while the secondary fastening means of prior art requires the user to be detailed instructed how to use the specific fastening means.

Further developments of the present invention are described in the dependent claims 2 to 13.

One embodiment of the invention will hereinafter be described by reference to the figures, wherein:
Figure 1 is a fragmented top plan view, broken away, for a disposal diaper constructed according to an embodiment of the invention;
Figure 2 is a sectional view taken along line 2-2 on Figure 1;
Figure 3 is a sectional view taken along line 3-3 on Figure 1;
Figure 4 is a perspective view of the diaper of Figure 1 folded and fastened by slotted tabs on the ends of the rear waistband which are interlocked securing the diaper for disposal; and
Figure 5 is an enlarged partial perspective view of the diaper of Figure 4 illustrating the slotted tabs being placed in their locked position for securing the rolled diaper for disposal.

The absorbent article shown on the drawings utilizes a first fastener means of the mechanical type positioned at one of the longitudinal ends of the garment which comprise one waistband portion of the article. The other cooperating waistband portion is at the opposite longitudinal end of the garment; the two waistband portions encircling the waist of the user in overlapping fashion. The mechanical type fastener illustrated includes two cooperating components mounted on the waistband portions that interengage one another in the overlapping of the waistband portions to releasably fasten the waistband in an adjusted, snug fit on the wearer. One component is a hook style swatch or tab and the second component is a loop style patch.

In the present invention, a second fastener means is included and positioned on the article to enable rolling or folding the absorbent article after use into a neat bundle for subsequent handling and disposal.

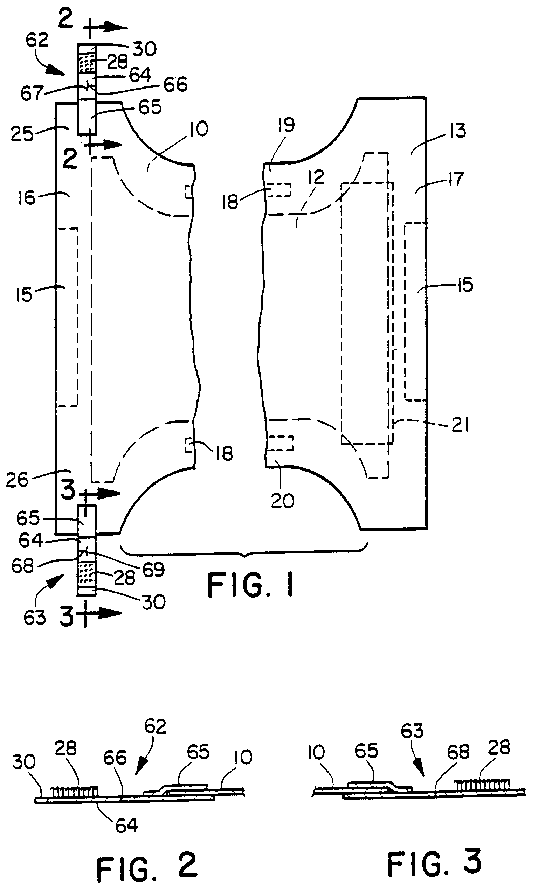

Referring to Figures 1 to 3, an absorbent article is constructed which has a primary mechanical fastener for the waistband of the article and a secondary fastener providing the disposability feature. The article includes an elongated impermeable backsheet 10 constructed of a layer of 25,4 µm (1 mil) polyethylene and TiO₂ filler which is overlaid with absorbent body 12, such as an absorbent body comprising 78% airlaid cellulose fiber and 11% hydrogel, enclosed in a 9% tissue wrap. The absorbent body has a density on the order of 0.12 grams per cubic centimeter. The absorbent body 12 is overlaid with a liquid permeable topsheet 13 corresponding in shape to backsheet 10. An example of the topsheet is 25,4 g/m² (0.75 ounce per square yard) polypropylene spunbond material. The backsheet 10 and topsheet 13 are fastened together peripherally by a spray application of adhesive, such as National 70-3016 adhesive, in order to form a composite. The tissue wrap is likewise adhesively attached to the inside of backsheet 10.

A suitable hook material may, for example, be produced from a process of continuous injection molding of a polymeric material, such as a polypropylene copolymer. It has been determined that the proper stiffness of the material is obtained from the copolymer having flexural modulus of 483 to 828 N/mm² (70,000 - 120,000 psi) and shore hardness value within the range of about D-40 to D-80, preferably about D-61.

The base strata of the hook material is fabricated onto the surface of the tab 30 by adhesive, heat-bonding or sonic-welding to provide the J-shaped hooks in a configuration facing outwardly of the tab structure. The hooks are tapered base to top with an alternating hook design. A specific example of a suitable hook is the HTH #707 available from Velcro U.S.A. The overall thickness (caliper) of the hook material is in the range of 0,089 - 0,13 cm (0.035 - 0.050 inch) and preferably about 0,11 cm (0.045 inch). The hooks are attached to a base film which is in the range of 0,013 - 0,064 cm (0.005 - 0.025 inch) thick, preferably a thickness in the range of about 0,0203 - 0,0254 cm (0.008 to 0.010 inch). The hook density on the base is within the range of 68 - 161 hooks per cm² (440 - 1040 hooks per square inch); preferably a hook density of about 114 hooks per cm² (740 hooks per square inch), as a particular example, hook material having a row density within the range of about 7,9 - 23,6 rows to the lineal cm (20 - 60 rows to the lineal inch) of width, preferably a row density of about 16 rows per cm (40 rows per lineal inch). The material used may be clear or opaque with selected color of the material.

A representative loop component of the hook and loop fastener is a fabric material of raised loop construction in which the fabric is stabilized (loops are erect from the fabric's base) through napping. Additionally, the loop material may be thermoset to impart other properties, such as is set forth in US-A-3 475 926 and US-A-3 090 097.

Preferably, a material of polyolefin-based fibers or other synthetic fibers is used; an example of the fabric being a tricot polyester. More specifically, polyethylene terephthalate (PET) fiber mix in which about 15 - 35% of the fabric yarns are yarns composed of 1 - 15 filaments with the yarn having a linear density within the range of about 1,67 - 3,3 tex (15 -30d), and about 65 - 85% of the fabric yarns are yarns having about 10 - 30 filaments per yarn and having a yarn linear density within the range of about 3,3 - 5,6 tex (30 - 50d) comprises the fibers used in the fabric. Two bar warp knit construction is preferred in which courses are in the range of 8,27 - 16,14 per cm (21 - 41 per inch) and wales are in the range of 10,27 - 18,11 per cm (26 - 46 per inch). The surface of the fabric is napped. The thickness caliper is within the range of about 0,254 - 1,02 mm (0.01 - 0.04 inch), the preferred caliper being about 0,89 mm (0.035 inch). The basis weight of the fabric is in the range of about 34 to 102 g/m² (1.0 - 3.0 ounces per square yard); the preferred basis weight being about 54,4 g/m² (1.6 ounces per square yard). The fabric is printable or decoratable directly, or in the alternative may be laminated over a predecorated film base, whereby a print pattern shows through the loop material. Loop material is applied onto the front waistband portion 17 in a patch or swatch 21 with the loops erect and facing outwardly from the face of the backsheet 10. The extent of patch 21 may be varied and may take one of several geometric shapes, such as rectangular, irregular shape, diamond, triangle, circle, oval, chevron or the like. The loop material may also be configured as a plurality of patches. The plural patches (two or more) form a landing zone for the hook tabs which are composed of multiple, separate sections. The variation of shape or configuration of multiple patches will lend itself to desired function and size for fastening the waistband about the various sizes of users.

Waist elastic members 15 (shown schematically on Figure 1) are attached along the opposite waistband portions 16 to 17 at the opposite longitudinal ends of the article. Preferably, the members 15 are at the outside margins of the waistbands 16 and 17, respectively. The elastic members are arranged to gather and shirr the waistbands 16 and 17 of the garment to provide a seal about the waist of the wearer. Also, leg elastic members 18 are attached to the oposite side margins 19 and 20 of the article and arranged to gather and shirr these side margins to provide seals about the legs of the wearer.

A first fastener means is provided on the garment comprising a fastening patch of loop material 21. The loop material 21 is one of the two components of the primary fastener means, and it interengages a second component in the form of hook materials, to be presently described. Loop material 21 may comprise a woven or nonwoven material. Preferably, the loop material is a Guilford warp knit fabric, number 19902, 54 g/m² (1.57 ounces per square yard), made from polyester fiber. Loop patch 21 is, for example, centered on the longitudinal axis, and attached by adhesive to the outside surface of backsheet 10 just below and interiorly of the elastic member at waistband 17. Patch 21 may alternatively extend to the terminal edge of the waistband. The loop material patch 21 may be adhesively fastened using National 70-3016 hot melt adhesive available from National Starch Company, in Bridgewater, New Jersey. In normal use, waistband 17 is disposed at the front of the garment article and waistband 16 is the rear portion which includes the ear portions 25 and 26. Waistband 16 wraps around the back of the torso and overlaps onto the front waistband 17; however, the garment may be constructed for encircling the waist in the reverse order. For the sake of description and illustration only, the front and rear waistbands 17 and 16, respectively, are identified in accordance with the conventional arrangement and use of the garment.

At the end portion of the article opposite the patch 21, (in this case, at the rear waistband 16) two fastener tabs 62 and 63 are attached securely to the backsheet to extend outwardly from the opposite ear portions 25 and 26, (Figure 1), repsectively, of the garment. Tabs 62 and 63 are each constructed of a tape 64 composed, for example, of a plastic film substrate, such as a 0,11 mm (4.5 mil) polypropylene having TiO₂ filler. The tabs each have end segments of the film sandwiched between the backsheet 10 and topsheet 13 in the opposite ear portions 25 and 26 along the sides of the article and the film substrate is attached thereat by a hot melt adhesive, such as National 70-3016 hot melt adhesive. The secured overlap of the tape 64 with backsheet 10 and topsheet 13 is approximately 25,4 mm (1.0 inch) measured inwardly from the margin of the ear portion; however, the overlap may be from 6,35 - 101,6 mm (0.25 - 4.0 inches). A fastener component, such as a region of hook material 28, is incorporated onto the film substrate to face upwardly in the direction of topsheet 13. In the illustrated embodiment, the hook material 28 is attached to the film substrate with hot melt adhesive (National 70-3016 being a satisfactory adhesive for this purpose). In an alternative embodiment, the hook material may be integrally formed with the film substrate. Hook material 28 may comprise Velcro® hook "style 15" and may be composed of a polypropylene, such as Telcar 102 polypropylene distributed by Teknor Apex Co. of Pawtucket, Rhode Island, or Ferro polypropylene 9004N distributed by Horizon Polymers of Houston, Texas. A finger tab portion 30 is exposed at the free outboard end of the attaching tape 64 for ease in grasping the tab and releasing the hook component once it is affixed to the loop component.

The garment is placed on the body of the wearer and front waistband 17 is overlapped by rear waistband 16 bringing the ear portions toward each other to a firm or snug fit. The hook material 28 on tabs 62 and 63 are pressed onto and engage the loop material of patch 21 facing outwardly on the backsheet at the front waistband mid-portion. The primary hook and loop fastener of the type described for closing the waistband and holding it in service about the wearer should have properties of shear force in the range of 0,046 - 0,138 N/mm² (6.6 - 20 psi), and peel force in the range of 7,9 - 47,2 g/mm (200 - 1200 grams per lineal inch) of transverse width. A preferred peel force value is within the range of about 15,75 - 23,62 g/mm (400 - 600 grams per inch) width.

"Shear" is determined according to ASTM Designation: D3654-82, "Standard Test Method for Holding Power of Pressure-Sensitive Tapes", which is incorporated herein by reference, and subject to the following modifications: In the relation to the test, the closure is placed under an increasing load. The system being tested is a hook and loop closure system. (See 1. Scope). The apparatus should include an "INSTRON" or equivalent continuous rate of extension (CRE) tensile tester. (See 3. Apparatus). In carrying out the procedure (See 6. Procedure), test direction of the materials should be noted. The test materials are rolled five cycles (6,45 cm² or 1 sq. in.), where one cycle equals once in each direction. The hook material is clamped into the lower jaw of the Instron tensile tester. The engaged system (hook and loop) is pulled until failure. In doing the calculations (See 10. Calculations), the peak load is determined and recorded in grams.

"Peel" is determined according to ASTM Designation: D1876-72, "Standard Test Methods for Peel Resistance of Adhesives (T-Peel Test)".

4.1 No test panels are used; hook and loop materials are directly engaged and are not mounted on any other substrate unless specified. Test direction of the material should be noted. No panels are used. The engaged test materials are rolled five cycles; where one cycle equals once in each direction. The hook material is clamped into the upper jaw and the loop material is clamped into the lower jaw.

In the suitable hook and loop fastening system, the fastener has a total peel resistance of at least about 150 gm. and preferably has a total peel resistance of at least about 400 gm. The total shear force resistance is at least about 750 gm. and preferably is at least about 1000 gm. It should be readily recognized that a suitable fastening system will include a selected balance between the property of total peel resistance and the property of total shear force resistance. For example, a system with the lower values of peel resistance could be more suitable if the system also exhibited a higher total shear force resistance.

For the purposes of the present description, the total peel resistance value corresponds to the peel force determined in accordance with ASTM D1876-72 multiplied by the transverse width of engagement between the hook material and the loop material employed in the particular fastening system. Similarly, the total shear force resistance value corresponds to the shear stress determined in accordance with ASTM D3654-82 multiplied by the area of engagement between the hook material and loop material of the fastening system.

The tabs 62 and 63 are each constructed of a relatively heavy and rigid substrate tape 64 (Figures 2 and 3), such as a 51 µm (2.0 mil) polypropylene. The substrate 64 is adhesively bonded onto the outer surface of the backsheet 10 by hot melt adhesive of the type disclosed earlier herein. A second tape 65 is overlaid on the inside surface of backsheet 10 and adhesively attached at its extreme end onto the surface of substrate tape 64 and the balance is adhered onto the inside surface of either the backsheet 10 or the composite layers of the ear portion where the tab is affixed, (Figures 2 and 3). Hook material 28 is attached to the inside face of tape 64 by adhesive, such as National 70-3016 hot melt adhesive, placing the hook portions on tabs 62 and 63 near the outboard end of the tape 64. The hook material may be, for example, Velcro hook style 15 made of Ferro polypropylene 9004N, as previously described. In use, the ears 25 and 26 overlie the front waistband 17 of the garment. Tabs 62 and 63 are pressed onto the loop material patch 21 securing the diaper about the waist of the wearer. This comprises the primary fastening of the diaper.

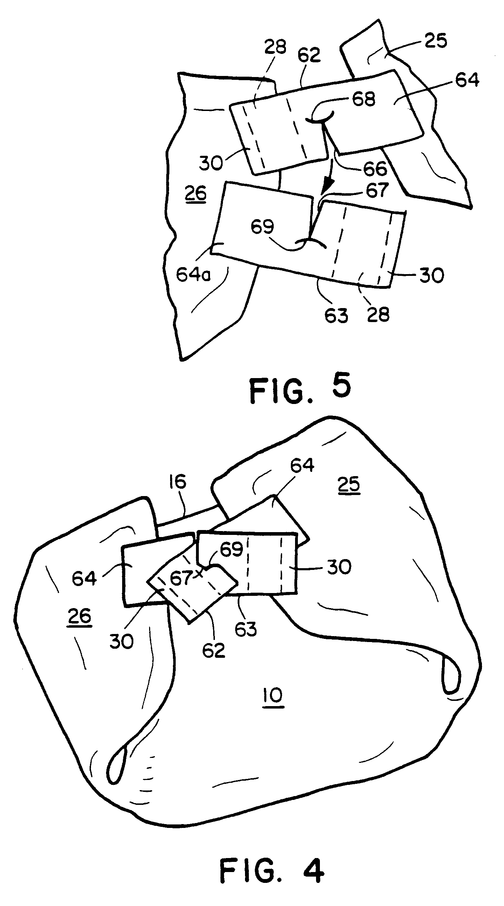

The secondary fastening means of the diaper for its disposal comprises a mechanical fastener. Tape 64, 64a of the tabs 62 and 63 are provided with a pair of interlocking slot fastener means between the hook tab portion 28 and the edge of the ear portion of the diaper. The tab 62 has a lateral slot 66 extending partially across the tape 64 and is flared angularly at 68 extending in opposite directions along the tape to provide a first Y-shaped slot element. The width of slot 66 is substantially the same as the thickness of tape 64. Similarly, tab 63 has a transverse slot 67, cut partially across tape 64a but from its opposite side edge in relation to the slot 66 of tab 62. The bottom of slot 67 has the oppositely flared angular ends 69 to form the second Y-shaped slot element. The Y-shaped slots 66, 68 and 67, 69 face in opposite longitudinal directions of the garment (Figure 1). Referring to Figures 4 and 5, the garment is disposed of after use by folding or rolling it from the front waistband 17 toward rear waistband 16. The opposite ears 25 and 26 of the diaper are folded inwardly toward each other placing the opening of the slots 66 and 67 opposite each other. By somewhat flexing the tab in arcuate form, the slots open up at the end (shown exaggerated on figure 5) for ease of interlocking them. The tabs 62 and 63 are brought together to engage the Y-shaped slots in each other to straddle the tapes until seated in the manner shown on Figure 4. The flared interior roots 68 and 69 of the two Y-shaped slots 66 and 67 interengage along the slot perimeter to lock the tabs 62 and 63 together. This provides a secondary fastening means for retaining the rolled, used garment for disposal.

In the disclosure, the elements are disclosed as being attached by adhesives, as the preferred examples for connecting the various fastener elements to the sheets and tapes of the article. It should be understood these attachments may be made by sonic welding, ultrasonic techniques, heat bonding (where applicable) or other known means of attachment.

In selecting a style of mounting the attachment tab 64 onto the article, certain trade-offs are available comparing security or strength of the attachment versus cost in adhesives, material and manufacturing cost of application.

In connection with the embodiments described herein and within the concept of the invention, the hook and loop mechanical fastener can be replaced with other mechanical fastening systems (not shown), such as buttons, hooks, hook and eye fasteners, snaps, or the like; cooperating with corresponding buttonholes, eye receivers or the like. While the representative embodiments of the invention described above include specific locations for the hook components and the loop components of the fastening systems, it is readily apparent that the relative locations of the hook and loop components may be reversed or exchanged for each other. In optional arrangements, a hook component may be substituted for the described loop component, and a loop component may be substituted for the described hook component to produce an alternative, operative configuration.

## Claims

1. An absorbent article, comprising:
superposed backsheet (10), absorbent body (12) and topsheet (13) elements formed to a composite,
the opposite longitudinal ends of said composite providing front and rear waistband portions (16,17) adapted to overlap each other upon encircling the abdomen of the user,
a primary fastening means (21,28) on said front and rear waistbands (16,17), respectively, for releaseably fastening the front and rear waistband portions (16,17) to each other in said encircling relationship; and
a separate secondary fastening means (62,63,66,67) on said composite for fastening the article in a rolled or folded condition for disposal after use, said secondary fastening means comprising tabs (62,63) projecting outwardly on the ends of the rear waistband (16),
**characterised in that**
said secondary fastening means further comprises transverse slits (66,67) across a portion of each said tabs (62,63), the slits (66,67) extending opposite each other in the respective tabs (62,63), whereby interengagement of the tabs (62,63) with each other at their said slits (66,67) fastens the article for disposal.

2. The article of claim 1 wherein the said slits (66,67) extend across a major portion of each of the tabs (62,63) and are a Y-shaped configuration inwardly of said tabs (62,63).

3. The article of claim 1 or 2 in which the primary fastener means comprises:
a mechanical hook and loop fastener comprising two interlocking materials (21,28), one being a hook material and the other being a loop material;
one of said two materials (28) being attached to the lateral end portions of said first waistband portion (16) and the other of said two materials (21) being attached to said second waistband portion (17), the said materials (21,28) being engageable to attach the first and second waistband portions (16,17) to each other.

4. The absorbent article of claim 3 in which the hook material (28) is attached on the first waistband portion (16) by laterally extending tapes (64) and a patch (21) of the loop material is attached on the second waistband portion (17) by adhesive.

5. An article as recited in claim 3 or 4, wherein said hook material comprises a polymeric base material of 0,0203 - 0,0254 cm thickness having a flexural modulus of 483 - 828 N/mm², a Shore hardness of about D-40 - D-80, and the hook configurations thereof being tapered base to top, and having a caliper in the range of 0,089 - 0,13 cm (0,035 - 0,050 inch).

6. The article as recited in any one of claims 3 to 5, wherein the hook design is tapered base to top with the tips of the hooks rounded with a radius, and wherein the hook material has a total caliper in the range of 0,089 to 0,130 centimeters, a hook density within the range of 68,2 to 161,2 hooks per square centimeter, a row density within the range of about 7,87 to 23,6 rows per centimeter, and a base film with a thickness within the range of about 0,0203 - 0,0254 centimeter.

7. An article as recited in any one of claims 3 to 6, wherein said loop material comprises a knit fabric composed of fabric yarns, about 15% - 35% of the fabric yarns having 1 - 15 filaments per yarn and a yarn linear density within the range of about 1,67 - 3,3 tex, and about 65% - 85% of the fabric yarns having 10 - 30 filaments per yarn and a yarn linear density within the range of about 3,3 - 5,6 tex.

8. The article as recited in claim 7, wherein said fabric is a two-bar warp knit fabric in which the courses of the warp knit are 8,27 - 16,1 per centimeter and the wales of said warp knit are 10,2 - 18,1 per centimeter.

9. The article as recited in claim 7 or 8, wherein the thickness caliper of said fabric is in the range of about 0,254 to 1,02 mm.

10. The article as recited in any one of claims 7 to 9, wherein the basis weight of said fabric is in the range of about 34 to 102 g/m².

11. The article as recited in any one of claims 7 to 10, wherein said fabric is a polyethylene-terephthalate tricot fabric of raised loop construction and the basis weight of the fabric is approximately 54,4 g/m² (1,6 ounces per square yard).

12. The article of any one of claims 1 to 11, wherein said primary fastening means (21,28) comprises a first and a second fastening component (21,28), said first fastening component (28) being attached to the tabs (62,63) of said secondary fastening means.

13. The article of any one of claims 1 to 12, **characterised by** being formed as a disposable diaper.

## Patentansprüche

1. Absorbierender Artikel mit:
Elementen einer rückwärtigen Lage (10), eines absorbierenden Körpers (12) und einer oberen Lage (13), die übereinanderliegend einen Verbund bilden,
wobei die gegenüberliegenden Enden des Verbunds in Längsrichtung vordere und rückwärtige Taillenbandbereich (16, 17) bilden, die derart ausgebildet sind, daß sie einander überlappen, nachdem sie um den Körper des Benutzers herumgeführt wurden,
einer primären Verschlußeinrichtung (21, 28) an jeweils den vorderen und den rückwärtigen Taillenbändern (16, 17) zum lösbaren Befestigen der vorderen und rückwärtigen Taillenbandbereich (16, 17) aneinander in der herumgeführten Beziehung; und
eine getrennte, zweite Verschlußeinrichtung (62, 63, 66, 67) am Verbund zum Verschließen des Artikels in einem aufgerollten oder gefalteten Wegwerfzustand nach Benutzung, wobei die sekundäre Verschlußeinrichtung Laschen (62, 63) enthält, die an den Enden des rückwärtigen Taillenbandes (16) nach außen vorstehen, **dadurch gekennzeichnet,** daß die sekundäre Verschlußeinrichtung ferner querverlaufende Schlitze (66, 67) über einen Bereich jeder Lasche (62, 63) umfaßt, wobei sich die Schlitze (66, 67) in den entsprechenden Laschen (62, 63) gegenläufig zueinander erstrecken, wobei ein gegenseitiger Eingriff der Laschen (62, 63) an ihren Schlitzen (66, 67) den Artikel zum Wegwerfen verschließt.

2. Artikel nach Anspruch 1, wobei die Schlitze (66, 67) sich über einen hauptsächlichen Bereich jeder der Laschen (62, 63) erstrecken und Y-förmig einwärts der Laschen (62, 63) geformt sind.

3. Artikel nach Anspruch 1 oder 2, bei dem die primäre Verschlußeinrichtung umfaßt:
einen mechanischen Haken- und Schlaufenverschluß mit zwei ineinandergreifenden Materialien (21, 28) von denen das eine ein Hakenmaterial und das andere ein Schlaufenmaterial ist;
wobei eines der beiden Materialien (28) an den seitlichen Endbereichen des ersten Taillenbandbereiches (16) und das andere der beiden Materialien (21) am zweiten Taillenbandbereich (17) angeordnet ist, wobei die besagten Materialien (21, 28) miteinander in Eingriff bringbar sind, um die ersten und zweiten Taillenbandbereiche (16, 17) aneinander zu befestigen.

4. Absorbierender Artikel nach Anspruch 3, wobei das Hakenmaterial (28) am ersten Taillenbandbereich (16) durch sich seitlich erstreckende Bänder (64) angeordnet ist, und wobei ein Flicken (21) des Schlaufenmaterials am zweiten Taillenbandbereich (17) durch einen Klebstoff befestigt ist.

5. Artikel nach Anspruch 3 oder 4, wobei das Hakenmaterial ein polymeres Basismaterial mit 0,0203 bis 0,0254 cm Dicke mit einem Biegemodul von 483 bis 828 N/mm², einer Härte nach Shore von etwa D-40 bis D-80 umfaßt, und wobei die Ausbildung seiner Haken von der Basis zur Spitze abgeschrägt ist, mit einem Kaliber im Bereich von 0,089 bis 0,13 cm (0,035 bis 0,050 Zoll).

6. Artikel nach einem der Ansprüche 3 bis 5, wobei die Hakengestaltung von der Basis zur Spitze abgeschrägt ist, wobei die Spitzen der Haken mit einem Radius abgerundet sind, und wobei das Hakenmaterial einen Gesamtkaliber im Bereich von 0,089 bis 0,130 cm, eine Hakendichte im Bereich von 68,2 bis 161,2 Haken pro Quadratzentimeter, eine Reihendichte innerhalb des Bereichs von 7,87 bis 23,6 Reihen pro Zentimeter und einen Grundfilm mit einer Dicke im Bereich von etwa 0,0203 bis 0,0254 cm aufweist.

7. Artikel nach einem der Ansprüche 3 bis 6, wobei das Schlaufenmaterial ein gewirktes Textilmaterial aufweist, bestehen aus Textilgarn, wobei etwa 15 % bis 35 % des Textilgarns 1 bis 15 Filamente pro Garn und eine lineare Garndichte innerhalb des Bereichs von etwa 1,67 bis 3,3 tex und etwa 65 % bis 85 % der Textilgarne 10 bis 30 Filamente pro Garn und eine lineare Garndichte im Bereich von etwa 3,3 bis 5,6 tex aufweisen.

8. Artikel nach Anspruch 7, wobei das Textilmaterial eine Kettenwirkware mit zwei Schußstreifen ist, bei der die Maschenreihen 8,27 bis 16,1 pro Zentimeter und die Maschenstäbchen 10,2 bis 18,1 pro Zentimeter betragen.

9. Artikel nach Anspruch 7 oder 8, wobei der Dickenkaliber des Textilmaterials im Bereich von etwa 0,254 bis 1,02 mm liegt.

10. Artikel nach einem der Ansprüche 7 bis 9, wobei das Grundgewicht des Textilmaterials im Bereich von etwa 34 bis 102 g/m² liegt.

11. Artikel nach einem der Ansprüche 7 bis 10, wobei das Textilmaterial ein Trikotstoff aus Polyäthylenterephthalat mit einer hochstehenden Schlaufenkonstruktion ist, und wobei das Grundgewicht des Textilmaterials etwa 54,4 g/m² (1,6 Unzen pro Quadratyard) beträgt.

12. Artikel nach einem der Ansprüche 1 bis 11, wobei die primäre Verschlußeinrichtung (21, 28) eine erste und eine zweite Verschlußkomponente (21, 28) aufweist, wobei die erste Verschlußkomponente (28) an den Laschen (62, 63) der sekundären Verschlußeinrichtung angeordnet ist.

13. Artikel nach einem der Ansprüche 1 bis 12, **gekennzeichnet durch** seine Ausbildung als Wegwerfwindel.

## Revendications

1. Article absorbant comprenant :
des élémentes superposée d'une feuille de renfort (10), d'un corps absorbant (12) et d'une feuille supérieure (13) constituant un composite,
les extrémités longitudinales opposées du dit composite fournissant des parties de ceinture avant et arrière (16, 17) adaptées pour se chevaucher l'une l'autre à l'endroit où elles encerclent l'abdomen de l'utilisateur,
des moyens de fermeture primaire (21, 28) respectivement situés sur lesdites ceintures avant et arrière, destinées à fermer de manière amovible les parties de ceinture avant et arrière (16, 17) l'une avec l'autre dans ladite relation d'encerclement; et
des moyens de fermeture secondaire (62, 63, 66, 67) situés sur ledit composite destinés à fermer l'article en condition d'enroulement ou de pliage pour que celui-ci soit jeté après utilisation, lesdits moyens de fermeture secondaire comprenant des languettes (62, 63) en saillie vers l'extérieur aux extrémités de la ceinture arrière (16),
caractérisé en ce que
lesdits moyens de fermeture secondaire comprennent en outre des encoches transversales (66, 67) au travers d'une partie de chacune des dites languettes (62, 63), les encoches (66, 67) s'étendant en opposition l'une par rapport à l'autre dans les languettes respectives (62, 63), l'engagement réciproque des languettes (62, 63) l'une avec l'autre au niveau de leurs dites encoches (66, 67) permettant la fermeture de l'article pour qu'il soit jeté.

2. Article selon la revendication 1 dans lequel lesdites encoches (66, 67) s'étendent au travers d'une partie plus importante de chacune des languettes (62, 63) et sont configurées en forme de Y vers l'intérieur des dites languettes (62, 63).

3. Article selon la revendication 1 ou la revendication 2 dans lequel les moyens de fermeture primaire comprennent :
une attache mécanique à crochets et à boucles comprenant deux matériaux s'entremêlant (21, 28), l'un étant un matériau à crochets et l'autre étant un matériau à boucles;
l'un des dits deux matériaux (28) étant fixé aux parties d'extrémité latérales de ladite première partie de ceinture (16) et l'autre des dits deux matériaux (21) étant fixé à ladite seconde partie de ceinture (17), lesdits matériaux pouvant s'engager pour fixer l'une à l'autre les première et seconde parties de ceinture (16, 17).

4. Article absorbant selon la revendication 3 dans lequel le matériau à crochets (28) est fixé sur la première partie de ceinture (16) par des bandes s'étendant latéralement (64) et une pièce (21) de matériau à boucles est fixée sur la seconde partie de ceinture (17) au moyen d'un adhésif.

5. Article selon la revendication 3 ou la revendication 4, dans lequel ledit matériau à crochets se compose d'un matériau de base polymère d'une épaisseur de 0,0203 à 0,0254 cm ayant un coefficient de réduction à la flexion de 483 à 828 N/mm², une dureté Shore d'environ D40 à D80, et les configurations de crochet de celui-ci sont coniques depuis la base vers le sommet et ont un calibre compris entre 0,089 et 0,13 cm (0,035 et 0,050").

6. Article selon l'une quelconque des revendications 3 à 5, dans lequel le dessin du crochet est conique depuis la base vers le sommet, les extrémités des crochets étant arrondies suivant un rayon, et dans lequel le matériau à crochets a un calibre total compris entre 0,089 et 0,130 cm, une densité de crochets comprise entre 682 et 161,2 crochets par cm², une densité de rangées comprise environ entre 7,87 et 23,6 rangées par centimètre, et un film de base dont l'épaisseur est comprise environ entre 0,0203 et 0,0254 centimètre.

7. Article selon l'une quelconque des revendications 3 à 6, dans lequel ledit matériau à boucles comprend un textile tricoté se composant de fils textiles, environ 15% à 35% des fils ayant 1 à 15 filaments par fil et une densité linéaire de fils comprise environ entre 1,67 et 3,3 tex, et environ 65% à 85% des fils textiles ayant 10 à 30 filaments par fil et une densité linéaire de fils comprise environ entre 3,3 et 5,6 tex.

8. Article selon la revendication 7, dans lequel ledit textile est un textile à mailles tricotées à deux barres dans lequel les rangées de mailles tricotées sont comprises entre 8,27 et 16,1 par centimètre et les colonnes des dites mailles sont comprises entre 10,2 et 18,1 par centimètre.

9. Article selon la revendication 7 ou la revendication 8, dans lequel le calibre d'épaisseur du dit textile est compris environ entre 0,254 et 1,02 mm.

10. Article selon l'une quelconque des revendications 7 à 9, dans lequel le poids de base du dit textile est compris environ entre 34 et 102 g/m².

11. Article selon l'une quelconque des revendications 7 à 10, dans lequel ledit textile est un textile tricoté en polythéréphthalate d'éthylène de fabrication à boucles relevées et le poids de base du textile est d'environ 54,4 g/cm² (1,6 once par yard carré).

12. Article selon l'une quelconque des revendications 1 à 11, dans lequel lesdits moyens de fermeture primaire (21, 28) comprennent un premier et un second composant de fermeture (21, 28), ledit premier composant de fermeture (28) étant fixé aux languettes (62, 63) des dits moyens de fermeture secondaire.

13. Article selon l'une quelconque des revendications 1 à 12, caractérisé en ce qu'il est formé en tant que couche jetable.
